# EUROPEAN PATENT APPLICATION

(11) **EP 4 046 598 A1**
(43) Date of publication of application: **24.08.2022**
(21) Application number: 20876448.0
(22) Date of filing: 18.09.2020
(51) Int. Cl.: A61F 2/16

(54) **ACCOMMODATING INTRAOCULAR LENS**

(30) Priority: 17.10.2019 JP 2019190327
(71) Applicant: Mirai Eye Inc., Hyogo 663-8113 (JP)
(72) Inventor: AKURA, Junsuke, Nishinomiya-shi, Hyogo 663-8113 (JP)
(74) Representative: Viering, Jentschura & Partner mbB Patent- und Rechtsanwälte
(86) International application number: PCT/JP2020/035585
(87) International publication number: WO 2021/075209

(57) **Abstract**

The present invention aims to provide an accommodating intraocular lens capable of capturing a minute movement of a lens capsule and amplifying it to a large curvature change in an optical portion. The intraocular lens includes an optical portion 31 and a support portion 32 arranged around the optical portion 31. The optical portion 31 has an anterior optical plate portion 311 and a posterior optical plate portion 312 to be arranged on the anterior and posterior sides of the lens capsule S and an optical body portion 313 arranged between the anterior optical plate portion 311 and the posterior optical plate portion 312. The anterior optical plate portion 311 and the posterior optical plate portion 312 are formed in a convex shape. The support portion 32 has an outer support plate portion 323, an anterior support plate portion 321 extending radially inwardly from an anterior end portion of the outer support plate portion 323 and connected to a peripheral portion of an anterior optical plate portion 311 of optical portion 31, and a posterior support plate portion 322 extending radially inwardly from a posterior end portion of the outer support plate portion 323 and connected to a peripheral portion of a posterior optical plate portion 312 of the optical portion 31. The anterior support plate portion 321 and the posterior support plate portion 322 are formed in such a manner as to extend obliquely radially inward and rearward or obliquely radially inward and forward.

## Description

### Technical Field

The present invention relates to an accommodating intraocular lens to be inserted into a lens capsule whose anterior capsule has been incised in ophthalmic surgery, such as, e.g., lens capsule extraction surgery to be performed as cataract surgery, refractive surgery, or presbyopia correction surgery.

### Background of the Invention

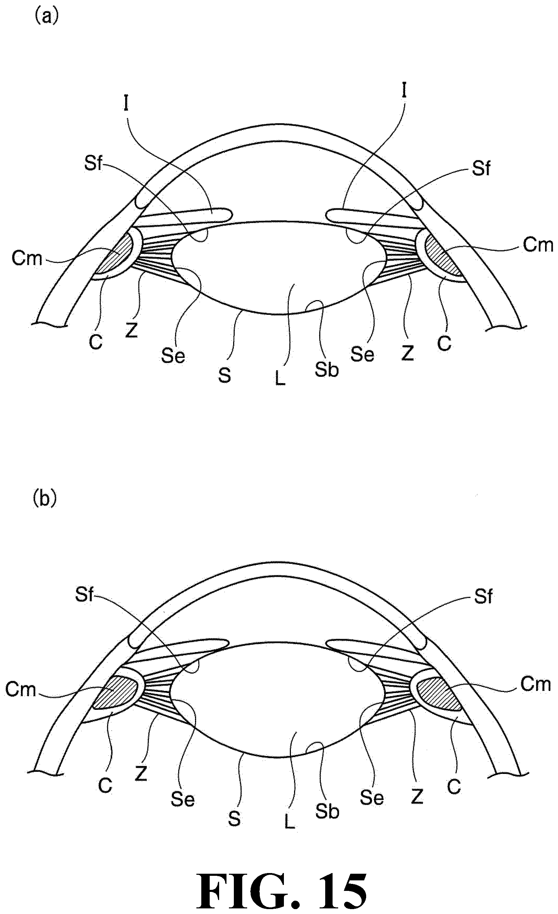

Usually, a focus adjustment of a human eye is performed by changing a thickness of a crystalline lens. A crystalline lens L is a convex transparent lens with a diameter of about 9 mm to 11 mm and a thickness of about 4 mm to 5 mm, as shown in FIG. 15. The crystalline lens L is fixed to the ciliary body C via the Zinn's zonule Z in a state of being wrapped by a transparent lens capsule S behind the iris I. The crystalline lens L adjusts the focal point by mainly changing the curvature of the front surface of the crystalline lens L in response to the movements of the ciliary body C during the adjustment.

A specific adjustment mechanism will be explained. For example, in a distance vision state, as shown in FIG. 15(a), the ciliary muscle Cm of the ciliary body C is relaxed. Therefore, the ciliary body C is in a position where the ciliary body C is retreated in a direction away from the lens capsule S. This state generates a relatively strong tension in the Zinn's zonule Z located between the ciliary body C and the equator Se of the lens capsule S. With this, the equator Se of the crystalline lens L is pulled radially outward, causing the reduction of the curvature of the anterior surface of the crystalline lens L in the lens capsule S to perform the focus adjustment at the time of the distance vision.

On the other hand, when an adjustment effort is made to look at a nearby object, as shown in FIG. 15(b), the ciliary muscle Cm of the ciliary body C contracts to cause the ciliary body C to protrude centripetally (in the direction toward the lens capsule equator Se). Thus, the ciliary body C is positioned in the direction close to the lens capsule S. As a result, the tension of the Zinn's zonule Z is weakened, increasing the curvature of the front surface of the crystalline lens L due to the inherent elasticity thereof to perform the focus adjustment at the time of the near vision.

As described above, the focus adjustment is performed by mainly changing the curvature of the anterior surface of the crystalline lens L to refract the light incident on the eye in accordance with the contraction and the relaxation of the ciliary muscle Cm of the ciliary body C. Note that in this adjustment mechanism, it is known that the contraction function and the relaxation function by the ciliary muscle Cm of the ciliary body C are kept relatively well even we get older. On the other hand, the cortices and the nuclei, which are the contents of the crystalline lens L, harden as we get older to lose flexibility, causing the curvature of the anterior surface of the crystalline lens L to become less likely to be changed. For this reason, it is known that the ability to freely adjust the focal point when shifting from the distance vision state to the near vision state is lost (this is called "presbyopia").

Incidentally, among diseases occurring in the above-described crystalline lens L, there is a disease called "cataract" in which the crystalline lens L becomes cloudy mainly due to aging. Many individuals have cataract surgery to treat the cataract. This surgery normally applies a method in which a circular hole is formed in the anterior capsule Sf, the turbid contents of the crystalline lens L are extracted via the hole by phacoemulsification, and an intraocular lens is inserted into the lens capsule S in a state in which only the transparent lens capsule S in the incised state remains. Cataract surgery has been currently administered to more than one million patients per year in Japan and more than three million patients per year in the United States of America. Various intraocular lenses used here have been proposed.

For example, the intraocular lens described in Patent Document 1 is referred to as a so-called "accommodating intraocular lens." The accommodating intraocular lens is provided with an optical portion (optical lens 42) and a support portion (optical lens positioning component 46). The support portion is provided with an anterior portion, a posterior portion and a curved portion connecting the anterior portion and the posterior portion. The optical portion and the anterior portion of the support portion are connected via haptic arms. With this, the support portion deforms in response to the movement of the lens capsule when in the distance vision state and in the near vision state to allow the movement of the optical portion in the anterior-posterior direction (see Patent Document 1).

### Prior Art Document

### Patent Document

Patent Document 1: Japanese Translation of PCT International Application Publication No. JP- T-2006-503661

### SUMMARY OF THE INVENTION

### Problems to be Solved by the Invention

However, the above-described accommodating intraocular lens is merely configured such that the support portion (particularly, the curved portion) is simply expanded or narrowed in the radial direction in response to the movement of the lens capsule. Therefore, the accommodating intraocular lens exhibits only a weak focus adjustment function, which is difficult to develop a practical focus adjustment function that allows the user to spend without glasses from reading to driving. For this reason, there has been a great need for an accommodating intraocular lens capable of capturing minute movements of the lens capsule caused by weak forces of shrinkage and relaxation of the ciliary muscle and amplifying the movements into relatively large movements of an optical portion to exert practical focus adjustment functions.

The present invention has been made in view of the technical background described above. It is an object of the present invention to provide an accommodating intraocular lens capable of capturing a minute movement of a lens capsule, amplifying the movement to a large change in the curvature of an optical portion to thereby exhibit a practical focus adjustment function.

### Means for Solving the Problem

The present invention relates to an accommodating intraocular lens to be placed in a lens capsule whose anterior capsule has been incised in surgery. The accommodating intraocular lens is provided with:
a deformable convex lens-shaped optical portion; and
one or a plurality of support portions arranged around the optical portion to support the optical portion.
The optical portion includes an anterior optical plate portion to be arranged on an anterior side in the lens capsule, a posterior optical plate portion to be arranged on a posterior side in the lens capsule, and an optical body portion arranged between the anterior optical plate portion and the posterior optical plate portion, at least the anterior optical plate portion being formed in a convex shape in which the anterior optical plate portion gently protrudes forward from a peripheral portion thereof toward a center portion thereof,.
The support portion includes an outer support plate portion to be arranged on a radially outward side in the lens capsule, an anterior support plate portion extending radially inward from an anterior end portion of the outer support plate portion, an inner end portion of the anterior support plate portion being connected to a peripheral portion of the anterior optical plate portion of the optical portion, and a posterior support plate portion extending radially inward from a posterior end portion of the outer support plate portion, an inner end portion of the posterior support plate portion being connected to a peripheral portion of the posterior optical plate portion of the optical portion, at least the anterior support plate portion being formed in such a manner as to extend diagonally rearward from the anterior end portion of the outer support plate portion to the peripheral portion of the anterior optical plate portion.
In a case where the lens capsule becomes a near vision state, when the outer support plate portion of the support portion is moved radially inward in accordance with a radially inward movement of an equator of the lens capsule, the inner end portion of the anterior support plate portion of the support portion is moved radially inward while approaching the posterior support plate portion, causing a radially inward movement of the peripheral portion of the anterior optical plate portion of the optical portion while approaching the posterior optical plate portion, which causes the optical portion to be deformed such that a central portion of the anterior optical plate portion of the optical portion bulges forward so as to increase curvature thereof to deform the optical body portion in such a manner that an anterior side of the optical body portion bulges forward to increase curvature thereof.
In a case where the lens capsule becomes a distance vision state, when the outer support plate portion of the support portion is moved radially outward in accordance with a radially outward movement of the equator of the lens capsule, the inner end portion of the anterior support plate portion of the support portion is moved radially outward while separating from the posterior support plate portion, causing a radially outward movement of the peripheral portion of the anterior optical plate portion of the optical portion while separating from the posterior optical plate portion, which causes the optical portion to be deformed such that the central portion of the anterior optical plate portion of the optical portion returns rearward so as to decrease the curvature thereof to deform the optical body portion in such a manner that the anterior side of the optical body portion returns rearward to decrease curvature thereof.

According to this, in a state in which the lens capsule is in a near vision state or in a distant state, when the outer support plate portion of the support portion is moved radially inward or radially outward in accordance with a radially inward or radially outward movement of the equator of the lens capsule, at least the inner end portion of the anterior support plate portion of the support portion is moved radially inward or radially outward while approaching or separating from the posterior support plate portion. In accordance with the movement, the periphery of the anterior optical plate portion of the optical portion moves radially inward or radially outward, which causes the central portion of the anterior optical plate portion of the optical portion to bulge or return while largely changing the curvature of the central portion of the anterior optical plate portion in the anterior-posterior direction. Thus, the anterior side of the optical plate portion can be bulged or returned while largely changing the curvature of the anterior side of the optical body portion in the anterior-posterior direction.

Further, it may be configured such that the support portion is formed in such a manner that the posterior support plate portion extends obliquely forward from a posterior end portion of the outer support plate portion to a peripheral portion of the posterior optical plate portion,
wherein in a case where the lens capsule becomes a near vision state, when the outer support plate portion of the support portion is moved radially inward in accordance with a radially inward movement of the equator of the lens capsule, an inner end portion of the posterior support plate portion of the support portion is moved radially inward while approaching the anterior support plate portion, causing a radially inward movement of the peripheral portion of the posterior optical plate portion of the optical portion while approaching the anterior optical plate portion, which causes the optical portion to be deformed such that a central portion of the posterior optical plate portion of the optical portion bulges rearward so as to increase curvature thereof to deform the optical body portion in such a manner that a posterior side of the optical body portion bulges rearward to increase the curvature thereof, and
wherein in a case where the lens capsule becomes a distance vision state, when the outer support plate portion of the support portion is moved radially outward in accordance with a radially outward movement of the equator of the lens capsule, the inner end portion of the posterior support plate portion of the support portion is moved radially outward while separating from the anterior support plate portion, causing a radially outward movement of the peripheral portion of the posterior optical plate portion of the optical portion while separating from the anterior optical plate portion, which causes the optical portion to be deformed such that a central portion of the posterior optical plate portion of the optical portion returns forward so as to decrease the curvature thereof to deform the optical body portion in such a manner that the posterior side of the optical body portion returns forward to decrease curvature thereof.

With the above-described configuration, in a state in which the lens capsule is in a near vision state or in a distant state, the outer support plate portion of the support portion is moved radially inward or radially outward in accordance with the radially inward or radially outward movement of the equator, the inner end portion of the posterior support plate portion of the support portion is moved radially inward or radially outward while approaching or separating from the anterior support plate portion. In accordance with the movement, the periphery of the posterior optical plate portion of the optical portion moves radially inward or radially outward, which causes the central portion of the posterior optical plate portion of the optical portion to bulge or retreat while largely changing the curvature of the central portion of the posterior optical plate portion in the anterior-posterior direction. Thus, the posterior side of the optical plate portion can be bulged or retreated while largely changing the curvature of the posterior side of the optical body portion in the anterior-posterior direction.

Further, the optical portion and the support portion may be configured such that the anterior optical plate portion of the optical portion and the anterior support plate portion of the support portion are integrally formed by a single plate member in a continuous manner, and the posterior optical plate portion of the optical portion and the posterior support plate portion of the support portion are integrally formed by a single plate member in a continuous manner. Further, it may be configured such that the optical portion and the support portion are configured separately in such a manner that an outer peripheral portion of the anterior optical plate portion and an outer peripheral portion of the posterior optical plate portion of the optical portion are sandwiched between the anterior support plate portion of the support portion and the posterior support plate portion of the support portion.

According to the above-described configuration, the central portion of the anterior optical plate portion of the optical portion can be bulged or retreated while largely changing the curvature in the anterior-posterior direction with a simple configuration.

Further, it may be connected such that the support portions are connected to each other along a circumferential direction by a deformable support portion connecting portion. With this, the accommodating intraocular lens can be stably reduced in diameter radially inward or increased in diameter radially outward, which allows the optical body portion to be stably deformed.

Further, it may be configured such that the support portion connecting portion is configured to be deformed in a curved shape protruding radially outward or radially inward when the optical portion and the support portion shift from a distance vision state to a near vision state to be reduced in diameter radially inward, and is configured to be deformed in a slightly curved shape extending in a peripheral direction or a linear shape when the optical portion and the support portion shift from a near vision state to a distance vision state to be increased in diameter radially outward. With this configuration, the accommodating intraocular lens can be more stably reduced in diameter radially inward or increased in diameter radially outward with the simple configuration. Further, in a case where the support portion connecting portion is in a curved shape protruding radially outward, a recess is formed between the support portion connecting portions, and therefore, it is possible to stably place a member for the support frame while fitting the member in the recess between the support plate connecting portions.

Further, the optical portion may be provided with a fluid material filled therein and a filling port for filling the fluid material. This makes it possible to simply and reliably fill the interior of the optical portion with the fluid material.

Further, the optical portion may be provided with a core member having rigidity at a central portion of the optical portion. With this, the optical portion can be stably deformed at a predetermined curvature.

Further, the accommodating intraocular lens may be further provided with:
a support frame to be placed in the lens capsule,
wherein the support frame is provided with:
   an anterior support frame portion to be arranged on an anterior side in the lens capsule to support the anterior capsule of the lens capsule;
   a posterior support frame portion to be arranged on a posterior side in the lens capsule to support a posterior capsule of the lens capsule; and
   one or a plurality of connecting portions connecting the anterior support frame portion and the posterior support frame portion, and
   wherein the support frame is configured to accommodate the optical portion and the support portion therein.

With this configuration, a slight movement of the lens capsule can be captured and assuredly transmitted to the support portion of the intraocular lens.

Further, the accommodating intraocular lens may be further provided with:
a lens capsule expansion bag to be placed in the lens capsule,
wherein the lens capsule expansion bag has an elastic force,
wherein the lens capsule expansion bag is provided with:
   an annular anterior bag portion to be placed in such a manner as to be in contact with the anterior capsule of the lens capsule;
   an annular posterior bag portion to be placed in such a manner as to be in contact with the posterior capsule of the lens capsule; and
   an annular intermediate bag portion protruded radially outward from outer peripheral portions of the anterior bag portion and the posterior bag portion, the annular intermediate bag portion being configured to be placed in such a manner as to be in contact with the equator of the lens capsule,
   wherein the lens capsule expansion bag is configured to accommodate the optical portion and the support portion therein.

This ensures that there is no gap between the lens capsule expansion bag and the equator of the lens capsule and that the equator of the lens capsule is constantly open in the anterior-posterior direction to assuredly prevent the lens capsule adhesion and the subsequent lens capsule cure due to the lens epithelial cell growth and fibrosis.

Further, a method of producing the accommodating intraocular lens includes the steps of:
preparing the support portion having a natural shape when the lens capsule is in a distance vision state and the optical portion having a natural shape when the lens capsule is in a near vision state;
forcibly reducing a diameter of the support portion radially inward as a whole such that the support portion shifts from the distance vision state to the near vision state;
joining the support portion and the optical portion while maintaining the near vision state of the support portion; and
deforming the optical portion from a state in which the curvature is large to a state in which the curvature is small by naturally expanding the support portion radially outward as a whole by an inherent restoring force of the support portion such that the support portion and the optical portion shift from the near vision state to the distance vision state.

With this, in a case where the accommodating intraocular lens is shifted from the distant vision state to the near vision state in the lens capsule, when the optical portion and the support portion are reduced as a whole radially inward by the lens capsule, the optical portion will attempt to return to its original natural shape, which enables assured deformation of the optical portion at a proper curvature. Further, in a state in which the accommodating intraocular lens is in a distance vision state, the support portion pulls the optical portion radially outward by its own restoring force, which enables stable deformation of the optical portion at an appropriate curvature.

### Effects of the Invention

According to the present invention, when the outer support plate portion of the support portion is moved radially inward or radially outward in accordance with the radially inward or radially outward movement of the equator in a state in which the lens capsule is in a near vision state or in a distance vision state, at least the inner end portion of the anterior support plate portion of the support portion is moved radially inward or radially outward while approaching or separating from the posterior support plate portion, causing the radially inward or radially outward movement of the peripheral portion of the anterior optical plate of the optical portion while approaching or separating from the posterior optical plate portion, which causes the central portion of the anterior optical plate portion of the optical portion bulges forward or retreats while changing the curvature of the central portion largely in the anterior-posterior direction. Therefore, at least the posterior side of the optical body portion can be bulged or retreated while largely changing the curvature in the anterior-posterior direction. For this reason, it is possible to capture the minute movements of the lens capsule to be converted into large changes in the curvature of the optical portion, which in turn can exert a practical focus adjustment function.

### BRIEF DESCRIPTION OF THE DRAWINGS

FIG. 1 is a perspective view of a lens capsule expansion bag of an accommodating intraocular lens according to this embodiment.
FIG. 2 is a cross-sectional view of the lens capsule expansion bag of FIG. 1.
FIG. 3 is a perspective view of a support frame of the accommodating intraocular lens according to this embodiment.
FIG. 4 is a cross-sectional view of the support frame of FIG. 3.
FIG. 5 is a perspective view of an intraocular lens body of the accommodating intraocular lens according to this embodiment.
FIG. 6 is a cross-sectional view of the intraocular lens body of FIG. 5.
FIG. 7 is a perspective view showing a state in which the intraocular lens body is placed in the support frame.
FIG. 8 is a cross-sectional view showing (a) a near vision state and (b) a distance vision state when the lens capsule expansion bag, the support frame, and the intraocular lens body are placed in a lens capsule.
FIG. 9 is a cross-sectional view showing a support frame and an intraocular lens body (separated members) according to another embodiment.
FIG. 10 is a cross-sectional view showing a support frame and an intraocular lens body (in which a core member is provided) according to still another embodiment.
FIG. 11 is a cross-sectional view showing a support frame and an intraocular lens body (in which a capsule member is provided) according to still yet another embodiment.
FIG. 12 is a perspective view showing a support frame and an intraocular lens body (in which support portion connecting portions are provided) according to still yet another embodiment.
FIG. 13 is a cross-sectional view showing a state in which an intraocular lens body is deformed on three-dimensional data.
FIG. 14 is a diagram showing a bench test result of a curvature change (accommodative power) of an intraocular lens body.
FIG. 15 is a schematic cross-sectional view showing movements at the time of focus adjustments in a human eye.

### EMBODIMENTS FOR CARRYING OUT THE INVENTION

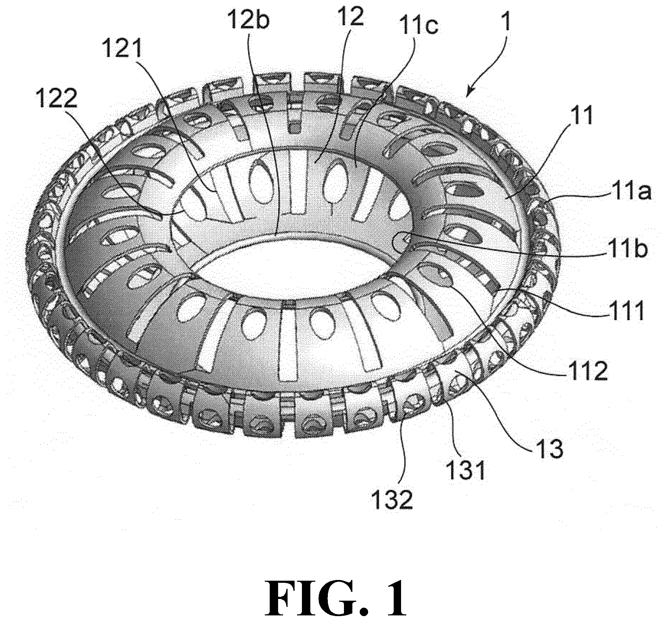

Next, some embodiments of an accommodating intraocular lens according to the present invention will be described with reference to FIG. 1 to FIG. 8. Note that the upper side in each drawing will be described as the anterior side (forward side) of a human eye, and the lower side will be described as a posterior side (rearward side) of a human eye.

The accommodating intraocular lens is provided with: a lens capsule expansion bag 1 to be placed in a lens capsule S whose anterior capsule Sf has been incised in ophthalmic surgery; a support frame 2 to be placed in the lens capsule expansion bag 1; and an intraocular lens body 3 to be placed in the support frame 2. Hereinafter, the configurations of the lens capsule expansion bag 1, the support frame 2, and the intraocular lens body 3 will be specifically described.

### [Lens Capsule Expansion Bag]

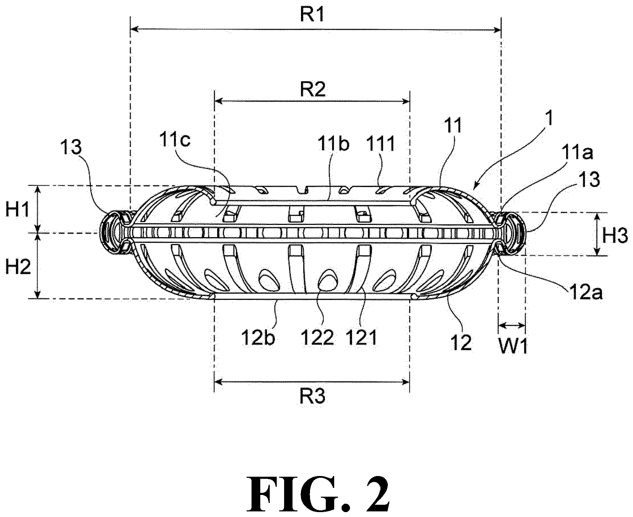

As shown in FIG. 1 and FIG. 2, the lens capsule expansion bag 1 is formed in a hollow doughnut shape as a whole by a flexible thin film or a plate-like elastic member (e.g., silicone). A ring-shaped insertion opening 11c for inserting a support frame 2 and the like is formed at the inner peripheral portion of the lens capsule expansion bag 1.

The lens capsule expansion bag 1 is provided with: an annular anterior bag portion 11 to be placed in such a manner as to be in contact with an anterior capsule Sf of a lens capsule S; an annular posterior bag portion 12 to be placed in such a manner as to be in contact with a posterior capsule Sb of the lens capsule S; and an annular intermediate bag portion 13 protruded radially outward from the outer peripheral portion of the anterior bag portion 11 and the outer peripheral portion of the posterior bag portion 12 and configured to be placed in such a manner as to be in contact with an equator Se of the lens capsule S. The anterior bag portion 11, the posterior bag portion 12, and the intermediate bag portion 13 are integrally molded by an elastic member as described above.

The anterior bag portion 11 is formed in a shape such that it extends radially outward from the outer peripheral portion 11a while gradually curving radially inward and forward, then extends while gradually curving radially inward and rearward, and reaches the radially inward inner peripheral portion 11b. Further, the anterior bag portion 11 is formed to have the outer diameter R1 of 9.6 mm, the inner diameter R2 of 5.0 mm, the height H1 of 1.2 mm, and the thickness (excluding the inner peripheral portion) of 0.09 mm.

Further, in the anterior bag portion 11, a linear opening 111 extending in the radial direction and an oval-shaped opening 112 extending in the radial direction are formed alternately along the circumferential direction. This makes it easier for the anterior bag portion 11 to deform in accordance with the movements of an anterior capsule Sf of a lens capsule S.

The posterior bag portion 12 is formed such that it extends radially outward from the outer peripheral portion 12a while gradually curving radially inward and rearward and then reaches the radially inward inner peripheral portion 12b. The posterior bag portion 12 is formed to have the outer diameter R1 of 9.6 mm, the inner diameter R3 of 5.0 mm, the height H2 of 1.7 mm, and the thickness (excluding the inner peripheral portion) of 0.09 mm.

Further, in the posterior bag portion 12, a linear opening 121 extending in the radial direction and an oval-shaped opening 122 extending in the radial direction are formed alternately along the circumferential direction. This makes it easier for the posterior bag portion 12 to deform in accordance with the movements of a posterior capsule Sb of a lens capsule S.

Further, the anterior bag portion 11 and the posterior bag portion 12 are each formed such that the thickness of the inner peripheral portion 11b, 12b is thicker than the thickness of a portion other than the inner peripheral portion 11b, 12b. As a result, the inner peripheral portion 11b, 12b is configured as a ring-shaped member having rigidity larger than the rigidity of a portion other than the inner peripheral portion 11b, 12b. Therefore, when the entire anterior bag portion 11 and the entire posterior bag portion 12 are deformed in accordance with the movements of a lens capsule S, the inner peripheral portion 11b of the anterior bag portion 11 and the inner peripheral portion 12b of the posterior bag portion 12 are each prevented from being inadvertently deformed in the radial direction. Therefore, the anterior bag portion 11 and the posterior bag portion 12 can be greatly deformed in response to small movements of a lens capsule S.

The intermediate bag portion 13 is formed in a shape such that it extends from the outer peripheral portion 11a of the anterior bag portion 11 while gently curving radially forward and outward, then extends while gently curving radially rearward and outward, then extends while gently curving radially rearward and inward, and then extends while gently curving radially forward and inward to reach the outer peripheral portion 12a of the posterior bag portion 12. The intermediate bag portion 13 is formed to have a radial width W1 of 0.7 mm, a height H3 of 1.2 mm, and a thickness of 0.06 mm.

As described above, the intermediate bag portion 13 is formed to have a diameter substantially equal to or slightly larger than the diameter of an equator Se of a lens capsule S. Thus, when placed in a lens capsule S, the intermediate bag portion 13 lightly presses the equator Se of the lens capsule S, causing a state in which there is no gap between the lens capsule expansion bag 1 and the equator Se of the lens capsule S at all times and that the equator Se of the lens capsule S is opened in the anterior-posterior direction more assuredly. This assuredly prevents the adhesion of the lens capsule S and the subsequent growth of lens epithelial cells and curing of the lens capsule S due to fibrosis.

Further, the intermediate bag portion 13 is formed such that the thickness thereof is thinner than the thickness of the anterior bag portion 11 and that of the posterior bag portion 12. Thus, the rigidity of the intermediate bag portion 13 is smaller than those of the anterior bag portion 11 and the posterior bag portion 12. As a result, the intermediate bag portion 13 is easily deformed in accordance with the movements of the equator Se of the lens capsule S.

Further, in the intermediate bag portion 13, a linear opening 131 extending in the anterior-posterior direction and three circular openings 132 arranged in the anterior-posterior direction are alternately formed along the circumferential direction. This makes it easier for the intermediate bag portion 13 to be deformed in accordance with the movements of the equator Se of the lens capsule S. When these openings 131 and 132 are formed by a laser, costs can be reduced, and the delivery time can be shortened.

### (Support Frame)

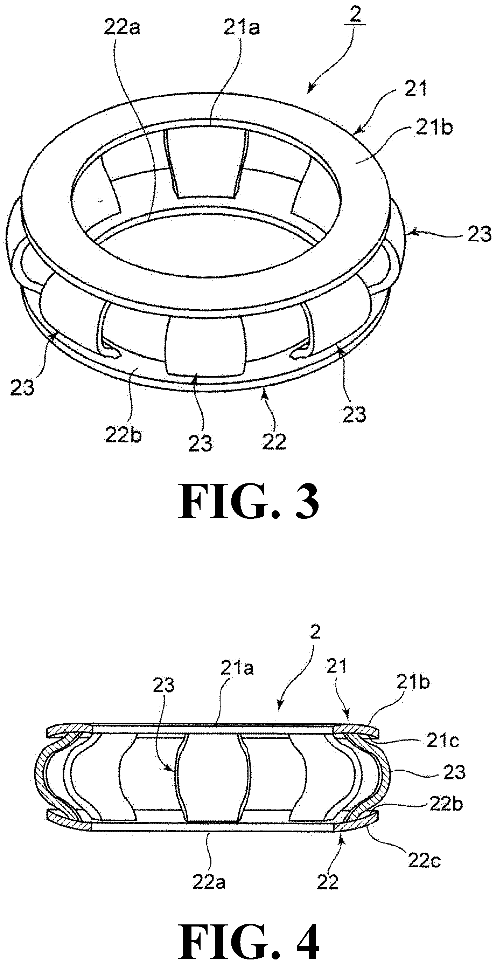

As shown in FIG. 3 and FIG. 4, the support frame 2 is provided with an anterior support frame portion 21 to be arranged on the anterior side in a lens capsule S, a posterior support frame portion 22 to be arranged on the posterior side in a lens capsule S, and a connecting portion 23 connecting the anterior support frame portion 21 and the posterior support frame portion 22.

The anterior support frame portion 21 is formed in an annular shape having an opening 21a at the central portion. When the anterior support frame portion 21 is placed in the lens capsule expansion bag 1 in a lens capsule S, the front surface 21b comes into contact with the inner surface of the lens capsule expansion bag 1 to indirectly press the anterior capsule Sf of the lens capsule S forward. Note that the anterior support frame portion 21 is made of an elastic material so as to be deformed somewhat in accordance with a force received from the anterior capsule Sf to further reduce the contact load on the anterior capsule Sf.

The posterior support frame portion 22 is formed in an annular shape having an opening 22a at the central portion. When the posterior support frame portion 22 is placed in the lens capsule expansion bag 1 in a lens capsule S, the rear surface 22c comes into contact with the inner surface of the lens capsule expansion bag 1 to indirectly press the posterior capsule Sb of the lens capsule S rearward. Note that the posterior support frame portion 22 is made of an elastic material so as to be deformed slightly in accordance with the force received from the posterior capsule Sb to further reduce the contact load on the posterior capsule Sb.

As shown in FIG. 3, the connecting portion 23 is composed of eight connecting pieces provided at regular intervals along the circumferential direction of the anterior support frame portion 21 and the posterior support frame portion 22. The connecting piece is formed of a thin plate-like member made of an elastic material, such as, e.g., a synthetic resin. One end of the connecting piece is fixed to the rear surface 21c of the anterior support frame portion 21 in such a manner as to extend radially outward. The other end of the connecting piece is fixed to the front surface 22b of the posterior support frame portion 22 in such a manner as to slightly extend radially outward. The intermediate portion of the connecting piece is formed to be bent in such a manner as to protrude radially outward.

Further, the connecting portion 23 connects the anterior support frame portion 21 and the posterior support frame portion 22 at a predetermined distance in a natural state in which the connecting piece is not elastically deformed. The predetermined distance is a length that causes the connecting piece to be bent slightly when the support frame 2 is placed in the lens capsule expansion bag 1.

Further, when the anterior support frame portion 21 and the posterior support frame portion 22 are moved in a direction of approaching each other in a state in which the support frame 2 is placed in the lens capsule expansion bag 1, the connecting portion 23 is bent in such a manner so as to expand radially outward, thereby generating a restoring force of returning the anterior support frame portion 21 and the posterior support frame portion 22 in the direction of separating each other. Therefore, the anterior support frame portion 21 and the posterior support frame portion 22 urge the anterior capsule Sf and the posterior capsule Sb by the restoring force of the connecting portion 23. This causes the anterior capsule Sf and the posterior capsule Sb to be extended in the anterior-posterior direction to open the equator Se of the lens capsule S, which can give a tension of moderate strength to the Zinn's zonule Z and the lens capsule S. In particular, in this embodiment, since a plurality of connecting pieces is arranged along the circumferential direction at equal intervals, the connecting portion 23 can extend and expand the anterior capsule Sf and the posterior capsule Sb uniformly along the entire circumference.

When the pressing force by the anterior bag portion 11 and the posterior bag portion 12 of the lens capsule expansion bag 1 with respect to the anterior support frame portion 21 and the posterior support frame portion 22 is decreased, the anterior support frame portion 21 and the posterior support frame portion 22 of the support frame 2 are moved away from each other, causing a radially inward movement of the connecting portion 23 of the support frame 2 while reducing the curvature of the central portion (curved portion) of the connecting portion 23.

On the other hand, when the pressing force by the anterior bag portion 11 and the posterior bag portion 12 of the lens capsule expansion bag 1 with respect to the anterior support frame portion 21 and the posterior support frame portion 22 is increased, the anterior support frame portion 21 and the posterior support frame portion 22 of the support frame 2 are caused to approach each other, causing a radially outward movement of the connecting portion 23 of the support frame 2 while increasing the curvature of the central portion (curved portion) of the connecting portion 23.

Note that a lens may be fitted to the opening 22a of the posterior support frame portion 22 of the support frame 2 to supplement the focus adjustment ability of the optical portion 31 to secure the refractive power of the accommodating intraocular lens as a whole according to a case.

### (Intraocular Lens Body)

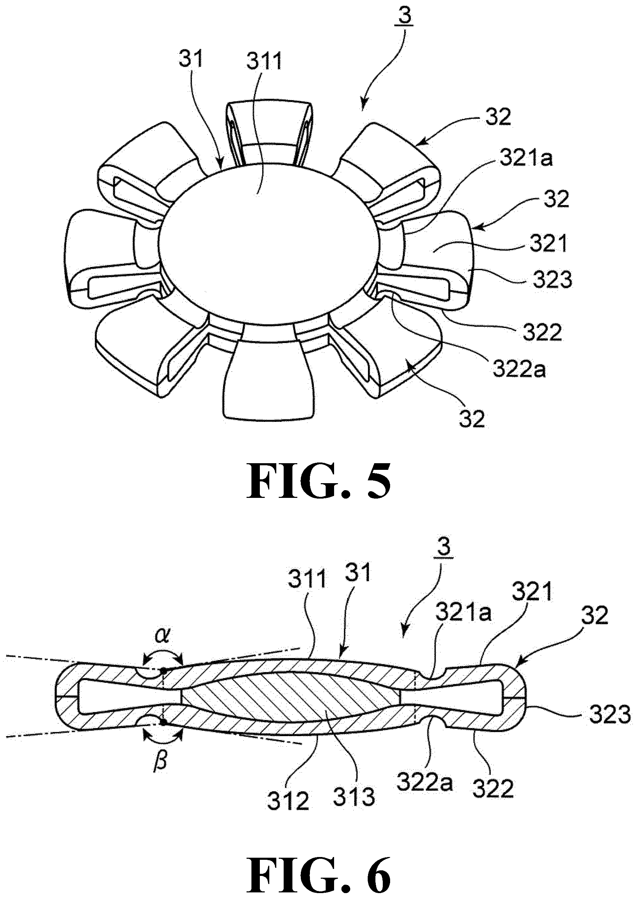

As shown in FIG. 5 and FIG. 6, the intraocular lens body 3 is provided with: a deformable convex lens-shaped optical portion 31 arranged at the central portion, the optical portion being made of synthetic resin or silicone; and eight support portions 32 arranged around the radially outer periphery of the optical portion 31, the support portion 32 being made of synthetic resin or silicone.

The optical portion 31 is configured by an anterior optical plate portion 311 to be arranged on the anterior side of a lens capsule S, a posterior optical plate portion 312 to be arranged on the posterior side of the lens capsule S, and an optical body portion 313 arranged between the anterior optical plate portion 311 and the posterior optical plate portion 312.

The anterior optical plate portion 311 is a disk-shaped plate member having predetermined rigidity with a thickness of about 0.15 mm and is formed in a convex shape that gently protrudes forward from the peripheral portion to the central portion. The posterior optical plate portion 312 is a disk-shaped plate member having predetermined rigidity with a thickness of about 0.15 mm and is formed in a convex shape that gently protrudes rearward from the peripheral portion to the central portion. These anterior optical plate portion 311 and posterior optical plate portion 312 are formed in the same size and shape with each other and are arranged substantially in parallel with each other with a predetermined gap in such a manner that the peripheral portions thereof are perfectly matched.

Note that when the anterior optical plate portion 311 and the posterior optical plate portion 312 are too thin like membranes or films, they become too soft to be deformed into an appropriate shape. On the other hand, when they are too thick, they become difficult to be deformed. For this reason, the thickness is set to 0.05 mm to 0.5 mm, preferably 0.08 mm to 0.4 mm, more preferably 0.1 mm to 0.2 mm in order to secure proper rigidity.

The optical body portion 313 is composed of an elastic gel-like member (e.g., silicone gel) and is arranged in such a manner as to be in close contact with the inner surfaces of the anterior optical plate portion 311 and the posterior optical plate portion 312 in the gap between the anterior optical plate portion 311 and the posterior optical plate portion 312. This optical body portion 313 has the elasticity to the extent of not causing the leakage from between the peripheral portions of the anterior optical plate portion 311 and the posterior optical plate portion 312 and capable of following the deformation of the anterior optical plate portion 311 and the posterior optical plate portion 312 in the anterior-posterior direction.

The support portion 32 is provided with an outer support plate portion 323 to be arranged on the radially outward side in a lens capsule S, an anterior support plate portion 321 to be arranged on the anterior side in the lens capsule S, and a posterior support plate portion 322 to be arranged on the posterior side in the lens capsule S.

The outer support plate portion 323 is a plate-like member having rigidity formed in such a manner as to extend in the anterior-posterior direction and is configured to be engaged with the connecting portion 23 of the support frame 2 as will be described later.

The anterior support plate portion 321 is a plate-like member having rigidity formed in such a manner as to extend linearly from the anterior end portion of the outer support plate portion 323 toward the radially inward diagonally posterior side. The anterior support plate portion 321 is continuously connected to the peripheral portion of the anterior optical plate portion 311 of the optical portion 31 by integral molding to constitute a single plate member together with the anterior optical plate portion 311.

The posterior support plate portion 322 is a plate-like member having rigidity formed in such a manner as to extend linearly from the posterior end portion of the outer support plate portion 323 toward the radially inward diagonally anterior side. The posterior support plate portion 322 is continuously connected to the peripheral portion of the posterior optical plate portion 312 by integral molding to constitute a single plate member together with the posterior optical plate portion 312.

When the intraocular lens body 3 receives a force radially inward from the periphery of the support portion 32, it shifts from a state in which the diameter is enlarged to a state in which the diameter is reduced radially inward. Specifically, when the outer support plate portion 323 of the support portion 32 is forcibly moved radially inward, the inner end portion of the anterior support plate portion 321 of the support portion 32 and the inner end portion of the posterior support plate portion 322 of the support portion 32 move radially inward while approaching each other. In accordance with the movement, the peripheral portion of the anterior optical plate portion 311 of the optical portion 31 and the peripheral portion of the posterior optical plate portion 312 of the optical portion 31 are moved radially inward while approaching each other, and the central portion of the anterior optical plate portion 311 of the optical portion 31 is deformed in such a manner as to protrude forward to increase the curvature and that the central portion of the posterior optical plate portion 312 of the optical portion 31 is deformed in such a manner as to protrude rearward to increase the curvature. At this time, the optical body portion 313 of the optical portion 31 is deformed into a steep convex shape while bulging in the anterior-posterior direction so as to follow the deformation of the anterior optical plate portion 311 and the posterior optical plate portion 312 in the anterior-posterior direction.

On the other hand, when the force received radially inward from the periphery of the support portion 32 is released, the intraocular lens body 3 shifts from a state in which the diameter is reduced radially inward to a state in which the diameter is expanded. Specifically, when the outer support plate portion 323 of the support portion 32 is moved from a state in which it is forcibly moved radially inward to return radially outward, the inner end portion of the anterior support plate portion 321 of the support portion 32 and the inner end portion of the posterior support plate portion 322 of the support portion 32 move radially outward while separating from each other. In accordance with the movement, the peripheral portion of the anterior optical plate portion 311 of the optical portion 31 and the peripheral portion of the posterior optical plate portion 312 of the optical portion 31 move radially outward while separating from each other, and the central portion of the anterior optical plate portion 311 of the optical portion 31 is deformed in such a manner as to return rearward to decrease the curvature and that the central portion of the posterior optical plate portion 312 of the optical portion 31 is deformed in such a manner as to return forward to decrease the curvature. At this time, the optical body portion 313 of the optical portion 31 is deformed into a gentle convex shape while returning in the anterior-posterior direction so as to follow the deformations of the anterior optical plate portion 311 and the posterior optical plate portion 312 in the anterior-posterior direction.

Further, in this embodiment, the anterior support plate portion 321 is provided with a recess 321a formed along the circumferential direction on the front surface of the connecting portion connecting the anterior optical plate portion 311 of the optical portion 31. Further, the posterior support plate portion 322 is provided with a recess 322a formed along the circumferential direction on the rear surface of the connecting portion connecting the posterior optical plate portion 312 of the optical portion 31. Therefore, the connecting portion between the support portion 32 and the optical portion 31 is likely to be bent, which makes the central portion of the anterior optical plate portion 311 of the optical portion 31 and the central portion of the posterior optical plate portion 312 of the optical portion 31 can be easily deformed in the anterior-posterior direction.

Note that the connection angle α between the anterior optical plate portion 311 and the anterior support plate portion 321 and the connection angle β between the posterior optical plate portion 312 and the posterior support plate portion 322 are preferably in the range of 135 degrees to 175 degrees, more preferably in the range of 145 degrees to 170 degrees, considering the efficient curvature deformation of the central portions of the anterior optical plate portion 311 and the posterior optical plate portion 312.

### (Method of Placing Accommodating Intraocular Lens)

Next, a method of placing the accommodating intraocular lens in a lens capsule S will be described.

First, the lens capsule expansion bag 1 is inserted into a lens capsule S whose anterior capsule Sf has been incised using an injector or tweezers in ophthalmic surgery. The lens capsule expansion bag 1 is placed such that the anterior bag portion 11 of the lens capsule expansion bag 1 comes into contact with the inner surface of the anterior capsule Sf of the lens capsule S, the posterior bag portion 12 comes into contact with the inner surface of the posterior capsule Sb of the lens capsule S, and the intermediate bag portion 13 comes into contact with the inner surface of the equator Se of the lens capsule S.

At this time, the lens capsule expansion bag 1 is formed such that the height in the anterior-posterior direction is slightly larger than the height of the lens capsule S in the anterior-posterior direction. Therefore, the lens capsule expansion bag 1 is slightly deformed in the anterior-posterior direction in the lens capsule S. By the elastic force of the lens capsule expansion bag 1, the anterior bag portion 11 comes into contact with the anterior capsule Sf so as to support the anterior capsule Sf, and the posterior bag portion 12 comes into contact with the posterior capsule Sb so as to support the posterior capsule Sb.

Next, the support frame 2 is inserted into the lens capsule expansion bag 1 placed in the lens capsule S using an injector or tweezers. The support frame 2 is installed such that the anterior support frame portion 21 comes into contact with the inner surface of the anterior bag portion 11 of the lens capsule expansion bag 1 and that the posterior support frame portion 22 comes into contact with the inner surface of the posterior bag portion 12 of the lens capsule expansion bag 1.

At this time, the anterior support frame portion 21 of the support frame 2 and the posterior support frame portion 22 of the support frame 2 press the anterior capsule Sf of the lens capsule S and the posterior capsule Sb of the lens capsule S, respectively. Therefore, the equator Se of the lens capsule S expands to extend the peripheral portion of the equator Se of the lens capsule S in the anterior-posterior direction. At the same time, the equator Se of the lens capsule S centripetally moves radially inward to reduce the diameter of the equator Se of the lens capsule S. This causes the Zinn's zonule Z to be pulled in both the direction toward the lens capsule S and the direction toward the ciliary body C, continually providing tension to the Zinn's zonule Z, thereby consequently providing tension to the lens capsule S. For this reason, it becomes a state in which the Zinn's zonule Z is ready to transmit the slight contraction and relaxation of the ciliary muscle Cm of the ciliary body C to the lens capsule S.

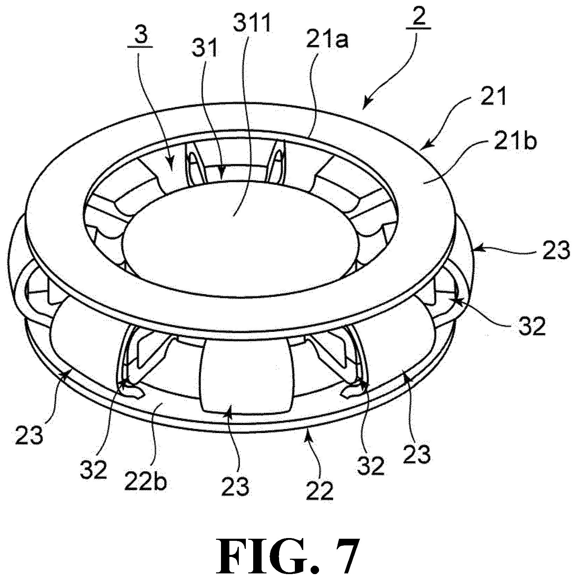

Next, the intraocular lens body 3 is inserted into the support frame 2 placed in the inside lens capsule expansion bag 1 using an injector or tweezers such that the support portion 32 (the outer support plate portion 323) is engaged with the inner surface of the connecting portion 23 of the support frame 2. Thereby, the optical portion 31 of the intraocular lens body 3 is placed in such a manner as to be perpendicular to the anterior-posterior direction at a height position near the equator Se of the lens capsule S (see FIG. 7).

In this embodiment, although the lens capsule expansion bag 1, the support frame 2, and the intraocular lens body 3 are placed in the lens capsule S in order, they may be placed in the lens capsule S in a state in which all or some of them are assembled in advance.

### (Operation of Accommodating Intraocular Lens)

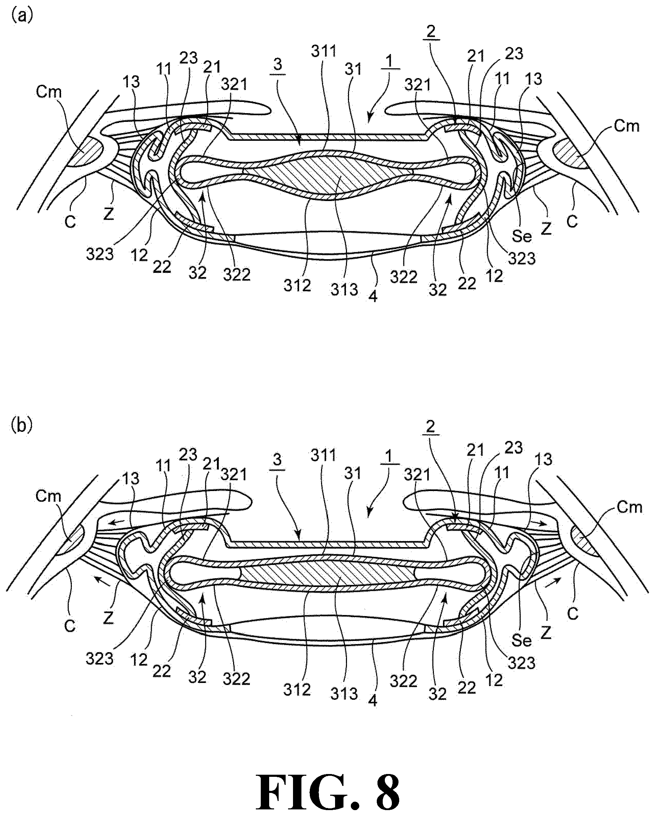

Next, referring to FIG. 8, the operation of the accommodating intraocular lens in the cases of a distance vision state and a near vision state will be described. In FIG. 8, (a) is a vertical cross-sectional view showing a near vision state of the accommodating intraocular lens, and (b) is a vertical cross-sectional view showing a distance vision state of the accommodating intraocular lens.

In a case where the lens capsule S shifts from a distance vision state to a near vision state, the ciliary muscle Cm of the ciliary body C relaxes to cause the ciliary body C to be positioned radially outward, as shown in FIG. 8 (b). This pulls the lens capsule S via the Zinn's zonule Z. Therefore, from the state in which the degree of the tension of the Zinn's zonule Z is increased (the distance vision state), as shown in FIG. 8(a), the ciliary muscle Cm of the ciliary body C contracts to bulge radially inward in the centripetal direction, resulting in a state in which the degree of the tension of the Zinn's zonule Z is lowered. This relaxes the tension of the peripheral portion of the equator Se of the lens capsule S, thereby lowering the pressing force to the anterior bag portion 11 and the posterior bag portion 12 by the anterior capsule Sf and the posterior capsule Sb.

Thus, the pressing force to the anterior support frame portion 21 of the support frame 2 by the anterior bag portion 11 and the pressing force to the posterior support frame portion 22 of the support frame 2 by the the posterior bag portion 12 are reduced. This causes the anterior support frame portion 21 of the support frame 2 of and the posterior support frame portion 22 of the support frame 2 to be distanced from each other, resulting in the reduced degree of curvature of the connecting portion 23, which causes the central portion of the connecting portion 23 to be moved radially inward.

When the outer support plate portion 323 of the support portion 32 is forcibly moved radially inward in accordance with the radially inward movement of the central portion of the connecting portion 23 of the support frame 2, the inner end portion of the anterior support plate portion 321 and the inner end portion of the posterior support plate portion 322 move radially inward while approaching each other. In accordance with the movement, the peripheral portion of the anterior optical plate portion 311 of the optical portion 31 and the peripheral portion of the posterior optical plate portion 312 of the optical portion 31 move radially inward. This causes the central portion of the anterior optical plate portion 311 of the optical portion 31 to be deformed such that the central portion of the anterior optical plate portion 311 bulges forward to increase the curvature. At this time, the optical body portion 313 of the optical portion 31 is deformed into a steep convex shape while bulging in the anterior-posterior direction so as to follow the deformation of the anterior optical plate portion 311 and the posterior optical plate portion 312 in the anterior-posterior direction.

On the other hand, in a case where the lens capsule S becomes from a near vision state to a distance vision state, as shown in FIG. 8(a), in a state in which the ciliary muscle Cm of the ciliary body C contracts and protrudes radially inward in a centripetal manner, and therefore, the degree of tension of the Zinn's zonule Z is reduced (in the near vision state), as shown in FIG. 8(b), the ciliary muscle Cm of the ciliary body C is relaxed to cause the ciliary body C to be positioned on the radially outward side. This pulls the lens capsule S via the Zinn's zonule Z, resulting in a state in which the degree of tension of the Zinn's zonule Z is increased. Therefore, the tension of the peripheral portion of the equator Se of the lens capsule S increases. Thus, the pressing force to the anterior bag portion 11 by the anterior capsule Sf increases, and the pressing force to the posterior bag portion 12 by the posterior capsule Sb increases.

Then, the pressing force to the anterior support frame portion 21 of the support frame 2 by the anterior bag portion 11 increases, and the pressing force to the posterior support frame portion 22 of the support frame 2 by the posterior bag portion 12 increases. As a result, the anterior support frame portion 21 of the support frame 2 and the posterior support frame portion 22 of the support frame 2 come close to each other, thereby increasing the degree of curvature of the connecting portion 23 of the support frame 2, which moves the central portion of the connecting portion 23 radially outward.

In accordance with the radially outward movement of the central portion of the connecting portion 23 of the support frame 2, when the outer support plate portion 323 of the support portion 32 moves radially outward from the state in which it is forcibly moved radially inward as the support portion 32 attempts to return to its natural state, the inner end portion of the anterior support plate portion 321 of the support portion 32 and the inner end portion of the posterior support plate portion 322 of the support portion 32 move radially outward while being separated from each other. In accordance with the movement, the peripheral portion of the anterior optical plate portion 311 of the optical portion 31 and the peripheral portion of the posterior optical plate portion 312 of the optical portion 31 are moved radially outward while being distanced from each other. Thus, the central portion of the anterior optical plate portion 311 of the optical portion 31 is returned rearward to be deformed such that the curvature is reduced. At the same time, the central portion of the posterior optical plate portion 312 of the optical portion 31 is returned forward to be deformed such that the curvature is reduced. At this time, the optical body portion 313 of the optical portion 31 is deformed into a gentle convex shape while returning in the anterior-posterior direction so as to follow the deformations of the anterior optical plate portion 311 and posterior optical plate portion 312 in the anterior-posterior direction.

As described above, the central portion of the anterior optical plate portion 311 of the optical portion 31 and the central portion of the posterior optical plate portion 312 of the optical portion 31 bulge or return while changing the curvature greatly in the anterior-posterior direction. For this reason, small movements of the lens capsule S can be captured and amplified to a large curvature change of the optical portion 31, which in turn can exert a practical focus adjustment function (10 diopters or more in experiments).

Note that in this embodiment, the lens capsule expansion bag 1 is placed in the lens capsule S, but it is not always required to be placed therein.

Further, although the support frame 2 is placed in the lens capsule expansion bag 1, it may not always be required to be placed therein. In this instance, the support portion 32 of the intraocular lens body 3 is engaged with the inner surface of the lens capsule expansion bag 1 or with the equator Se of the lens capsule S.

In addition, although the intraocular lens body 3 is provided with eight support portions 32, other numbers of the support portions 32 may be provided.

In the above-described intraocular lens body 3, both the anterior optical plate portion 311 and the posterior optical plate portion 312 are deformed in the anterior-posterior direction. However, only the anterior optical plate portion 311 may be deformed in the anterior-posterior direction.

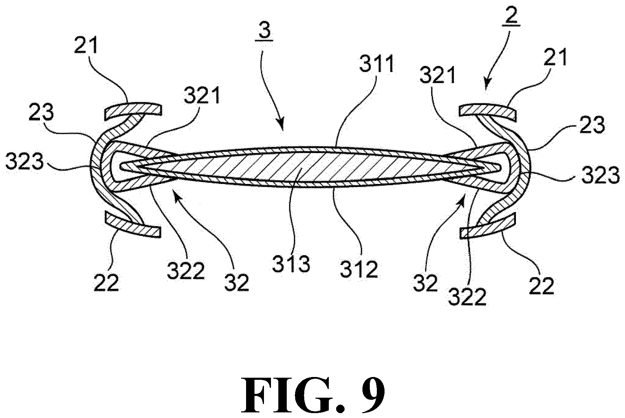

Further, the optical portion 31 and the support portion 32 are configured such that the anterior optical plate portion 311 of the optical portion 31 and the anterior support plate portion 321 of the support portion 32 are integrally formed in a continuous manner by a single plate member, and the posterior optical plate portion 312 of the optical portion 31 and the posterior support plate portion 322 of the support portion 32 are integrally formed in a continuous manner by a single plate member. However, as shown in FIG. 9, the optical portion 31 and the support portion 32 may be configured separately in such a manner that the outer peripheral portion of the anterior optical plate portion 311 of the optical portion 31 and the outer peripheral portion of the posterior optical plate portion 312 of the optical portion 31 are sandwiched between the anterior support plate portion 321 of the support portion 32 and the posterior support plate portion 322 of the support portion 32. At this time, the optical portion 31 and the support portion 32 may be joined together by an adhesive or the like, or may be further mechanically joined together by an uneven fitting or the like.

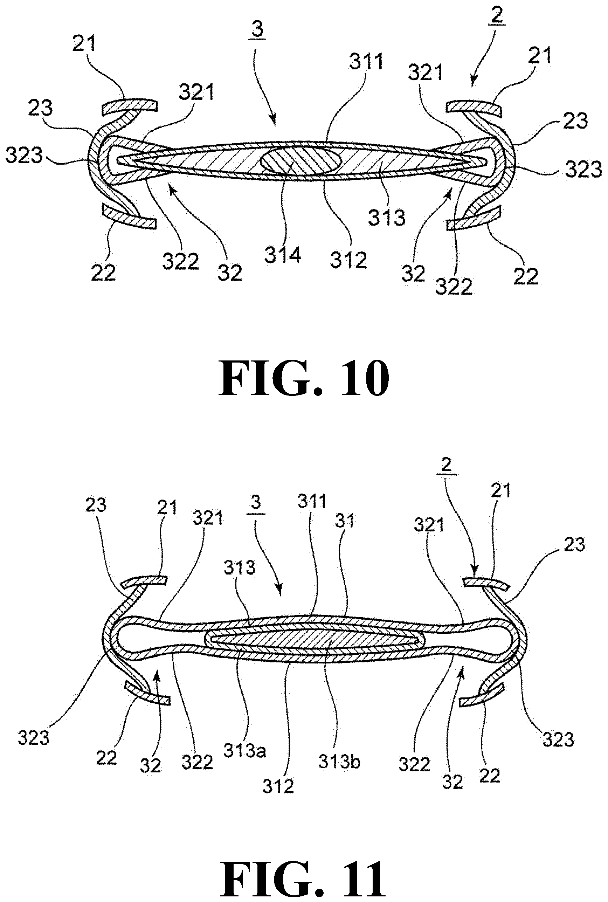

Further, as shown in FIG. 10, the optical body portion 313 may be provided with a core member 314 having rigidity at the central portion of the optical body portion 313.

Further, as shown in FIG. 11, it may be configured such that the optical body portion 313 is provided with an elastic capsule member 313a and a fluid material (gel, oil, etc.) 313b filled in the capsule member 313a and is arranged between the anterior optical plate portion 311 of the optical portion 31 and the posterior optical plate portion 312 of the optical portion 31.

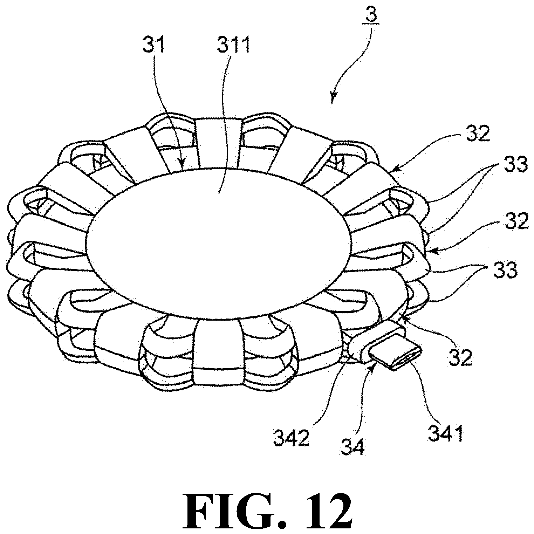

Further, as shown in FIG. 12, the support portions 32 may be connected to each other along the circumferential direction by a deformable support portion connecting portion 33. The support portion connecting portion 33 is not particularly limited in shape. As shown in FIG. 12, however, it may be configured such that when the intraocular lens body 3 is reduced in diameter by shifting from a distance vision state to a near vision state, the support portion connecting portion 33 is deformed into a curved shape protruding radially outward, while when the intraocular lens body 3 is increased in diameter by shifting from a near vision state to a distance vision state, the support portion connecting portion 33 is deformed into a gently curved shape or a linear shape extending in the circumferential direction. Note that in a case where the intraocular lens body 3 is reduced in diameter by shifting from a distance vision state to a near vision state, the support portion connecting portion 33 may be deformed into a curved shape protruding radially inward.

Further, as shown in FIG. 12, the optical body portion 313 may be provided with a filling port 34 for filling a flowable material. The filling port 34 is made of an elastic material, such as, e.g., silicone, and a slit 341 is formed by cutting in circumferential direction. To fill the inside of the optical body portion 313 with a fluid material, a tube (not shown) is inserted into the slit 341 of the filling port 34 to fill a fluid material. When the tube is pulled out from the slit 341 of the filling port 34, the slit is tightly closed by the elasticity of the silicone member or the like. Further, it may be configured such that a ring member 342 is fit around the filling port 34 to increase the adhesion degree of the slit 341 of the filling port 34 to thereby reliably prevent the leakage of the fluid material.

The anterior optical plate portion 311, the posterior optical plate portion 312, the optical body portion 313, and the support portion 32 are not particularly limited in material. For example, the anterior optical plate portion 311 and the posterior optical plate portion 312 may be made of silicone, polyurethane, acrylic, silicone hydrogel, and collamer. Further, the optical body portion 313 may be made of silicone oil, silicone gel, polyurethane gel, glycerin, or the like. Further, the support portion 32 may be made of silicone, polyurethane, acrylic, polyimide, polypropylene, or the like.

Further, as a method of producing the intraocular lens body 3, for example, the following Step can be exemplified.

### <Step 1>

A support portion 32 having a natural shape when a lens capsule S is in a distance vision state and an optical portion 31 having a natural shape when a lens capsule S is in a near vision state are prepared. At this time, as shown in FIG. 12, the support portions 32 are preferably coupled to each other along the circumferential direction by a deformable support portion connecting portion 33.

Note that "a support portion 32 having a natural shape when a lens capsule S is in a distance vision state" includes not only a case in which the support portion 32 has a complete natural shape when the lens capsule S is in a distance vision state but also a case in which the support portion 32 has a diameter that differs somewhat from the diameter of the natural shape when the lens capsule S is in a distance vision state. Further note that "an optical portion 31 having a natural shape when a lens capsule S is in a near vision state" includes not only a case in which the support portion 32 has a complete natural shape when the lens capsule S is in a near vision state but also a case in which the support portion 32 has a diameter that differs somewhat from the diameter of the natural shape when the lens capsule S is in a near vision state.

### <Step 2>

The support portion 32 is forcibly reduced in diameter radially inward as a whole in such a manner that the support portion 32 shifts from a distance vision state to a near vision state.

### <Step 3>

The support portion 32 and the optical portion 31 are joined while maintaining the near vision state of the support portion 32. For the joining of the support portion 32 and the optical portion 31, for example, as shown in FIG. 9, an embodiment can be exemplified in which the outer peripheral portion of the anterior optical plate portion 311 of the optical portion 31 and the outer peripheral portion of the posterior optical plate portion 312 of the optical portion 31 are sandwiched between the anterior support plate portion 321 of the support portion 32 and the posterior support plate portion 322 of the support portion 32.

### <Step 4>

The optical portion 31 is deformed from a state in which the curvature is large to a state in which the curvature is small by naturally expanding the support portion 32 as a whole radially outward by the inherent restoring force in such a manner that the support portion 32 and the optical portion 31 are shifted from a near vision state to a distance vision state.

According to this, when the optical portion 31 and the support portion 32 are reduced in diameter radially inward as a whole by the lens capsule S at the time of shifting the accommodating intraocular lens from a distance vision state to a near vision state in the lens capsule S, the optical portion 31 returns to its original natural shape. Therefore, the optical portion 31 can be assuredly deformed at an appropriate curvature. Further, in a distance vision state, the support portion 32 pulls the optical portion 31 radially outward by the inherent restoring force, it is possible to stably deform the optical portion 31 at an appropriate curvature.

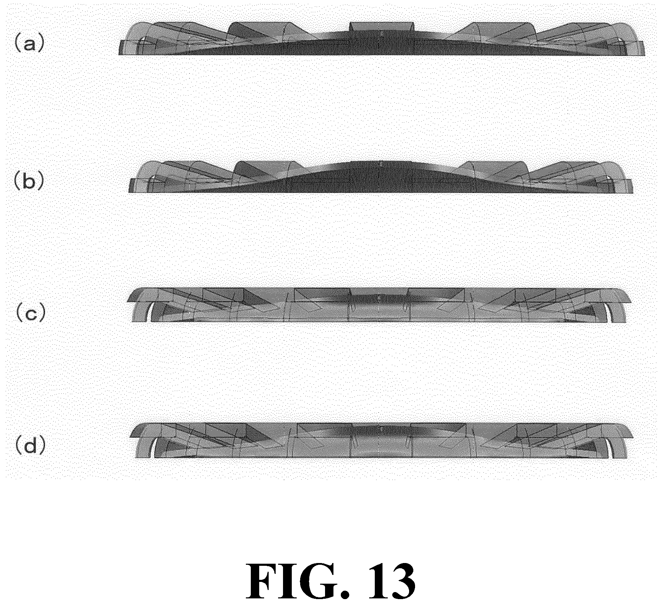

Further, FIG. 13 is a diagram showing experiments on three-dimensional data of the intraocular lens body 3. In FIG. 13, (a) and (b) show a case in which an oil with low viscosity is used as a fluid material of the optical body portion 313. In FIG. 13, (c) and (d) show a case in which a gel with relatively high viscosity is used as the fluid material of the optical body portion 313. In FIG. 13, (a) and (c) each show a distance vision state. In FIG. 13, (b) and (d) each show a near vision state. As is clear from these diagrams, it can be seen that the curvature of the anterior optical plate portion 311 of the optical portion 31 is greatly changed between the distance vision state and the near vision state.

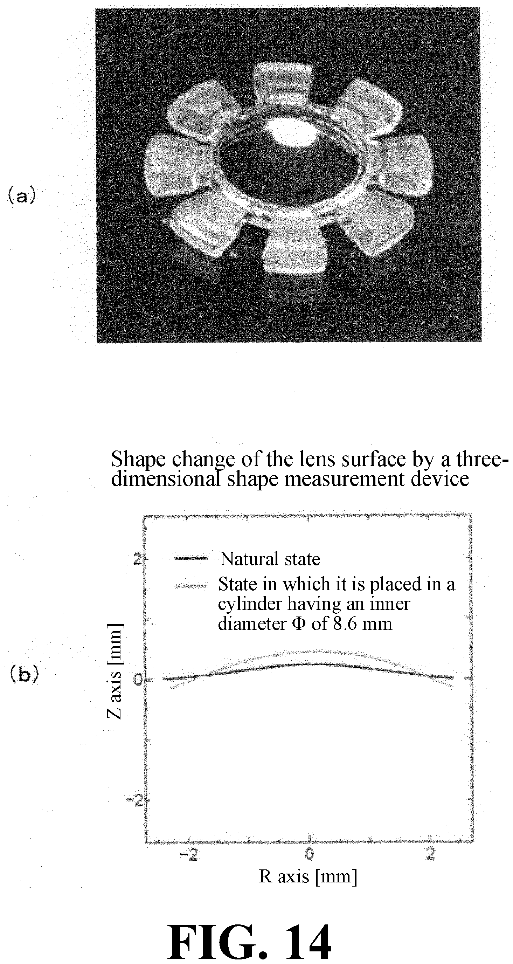

FIG. 14 shows a prototype of the intraocular lens body 3 and results of a bench test of a curvature change (accommodative power) of the anterior optical plate portion 311. The prototype of the intraocular lens body 3 has a diameter of 9 mm in a natural state and is provided with an anterior optical plate portion 311 made of silicone with a refractive index of 1.41 and an optical body portion 313 made of silicone gel with a refractive index similar to that of the anterior optical plate portion 311. In FIG. 14, (a) is a photograph of a prototype used in the bench test, and (b) is a graph of the bench test results (the horizontal axis indicates the diameter, and the vertical axis indicates the bulge height). As shown in FIG. 14(b), when the diameter of this entire prototype is reduced from 9 mm (natural state) to 8.6 mm (state in which it is placed in a cylinder having an inner diameter ϕ of 8.6 mm) with a jig, the curvature radius of the anterior optical plate portion 311 was changed from 8.2 mm to 4.4 mm, and the refractive power (accommodative power) of 7.8D was exerted. Similar bench tests revealed similar changes in the curvature radii for the posterior optical plate portion 312, indicating that the entire intraocular lens body 3 combined with the anterior optical plate portion 311 and the posterior optical plate portion 312 exhibits a refractive power change (accommodative power) of about 15.6D.

The embodiments of the present invention have been described above with reference to the attached drawings, but the present invention is not limited to the illustrated embodiments. It should be understood that various modifications and variations can be made to the illustrated embodiments falling within the same or equivalent scope as the present invention.

### Description of Symbols

- 1:: Lens capsule expansion bag
11: Anterior bag portion
11a: Outer peripheral portion
11b: Inner peripheral portion
11c: Insertion opening
111: Linear opening
112: Oval-shaped opening

- 12:: Posterior bag portion
12a: Outer peripheral portion
12b: Inner peripheral portion
121: Linear opening
122: Oval-shaped opening
13: Intermediate bag portion
131: Linear opening
132: Circular opening
2: Support frame
21: Anterior support frame portion
21a: Opening
21b: Front
21c: Posterior surface
22: Posterior support frame portion
22a: Opening
22b: Front
22c: Posterior surface
23: Connecting portion
3: Intraocular lens body
31: An optical portion
311: Anterior optical plate portion
312: Posterior optical plate portion
313: Optical body portion
313a: Capsule member
313b: Fluid material
314: Core material
32: Support portion
321: Anterior support plate portion
322: Posterior support plate portion
323: Outer support plate portion
33: Support portion connecting portion
34: Filling port

## Claims

1. An accommodating intraocular lens to be placed in a lens capsule whose anterior capsule has been incised in surgery, the accommodating intraocular lens comprising:
a deformable convex lens-shaped optical portion; and
one or a plurality of support portions arranged around the optical portion to support the optical portion,
wherein the optical portion includes an anterior optical plate portion to be arranged on an anterior side in the lens capsule, a posterior optical plate portion to be arranged on a posterior side in the lens capsule, and an optical body portion arranged between the anterior optical plate portion and the posterior optical plate portion, at least the anterior optical plate portion being formed in a convex shape in which the anterior optical plate portion gently protrudes forward from a peripheral portion thereof toward a center portion thereof,
wherein the support portion includes an outer support plate portion to be arranged on a radially outward side in the lens capsule, an anterior support plate portion extending radially inward from an anterior end portion of the outer support plate portion, an inner end portion of the anterior support plate portion being connected to a peripheral portion of the anterior optical plate portion of the optical portion, and a posterior support plate portion extending radially inward from a posterior end portion of the outer support plate portion, an inner end portion of the posterior support plate portion being connected to a peripheral portion of the posterior optical plate portion of the optical portion, at least the anterior support plate portion being formed in such a manner as to extend diagonally rearward from the anterior end portion of the outer support plate portion to the peripheral portion of the anterior optical plate portion,
wherein in a case where the lens capsule becomes a near vision state, when the outer support plate portion of the support portion is moved radially inward in accordance with a radially inward movement of an equator of the lens capsule, the inner end portion of the anterior support plate portion of the support portion is moved radially inward while approaching the posterior support plate portion, causing a radially inward movement of the peripheral portion of the anterior optical plate portion of the optical portion while approaching the posterior optical plate portion, which causes the optical portion to be deformed such that a central portion of the anterior optical plate portion of the optical portion bulges forward so as to increase curvature thereof to deform the optical body portion in such a manner that an anterior side of the optical body portion bulges forward to increase curvature thereof, and
wherein in a case where the lens capsule becomes a distance vision state, when the outer support plate portion of the support portion is moved radially outward in accordance with a radially outward movement of the equator of the lens capsule, the inner end portion of the anterior support plate portion of the support portion is moved radially outward while separating from the posterior support plate portion, causing a radially outward movement of the peripheral portion of the anterior optical plate portion of the optical portion while separating from the posterior optical plate portion, which causes the optical portion to be deformed such that the central portion of the anterior optical plate portion of the optical portion returns rearward so as to decrease the curvature thereof to deform the optical body portion in such a manner that the anterior side of the optical body portion returns rearward to decrease curvature thereof.

2. The accommodating intraocular lens as recited in claim 1,
wherein the support portion is formed in such a manner that the posterior support plate portion extends obliquely forward from a posterior end portion of the outer support plate portion to a peripheral portion of the posterior optical plate portion,
wherein in a case where the lens capsule becomes a near vision state, when the outer support plate portion of the support portion is moved radially inward in accordance with a radially inward movement of the equator of the lens capsule, an inner end portion of the posterior support plate portion of the support portion is moved radially inward while approaching the anterior support plate portion, causing a radially inward movement of the peripheral portion of the posterior optical plate portion of the optical portion while approaching the anterior optical plate portion, which causes the optical portion to be deformed such that a central portion of the posterior optical plate portion of the optical portion bulges rearward so as to increase curvature thereof to deform the optical body portion in such a manner that a posterior side of the optical body portion bulges rearward to increase the curvature thereof, and
wherein in a case where the lens capsule becomes a distance vision state, when the outer support plate portion of the support portion is moved radially outward in accordance with a radially outward movement of the equator of the lens capsule, the inner end portion of the posterior support plate portion of the support portion is moved radially outward while separating from the anterior support plate portion, causing a radially outward movement of the peripheral portion of the posterior optical plate portion of the optical portion while separating from the anterior optical plate portion, which causes the optical portion to be deformed such that a central portion of the posterior optical plate portion of the optical portion returns forward so as to decrease the curvature thereof to deform the optical body portion in such a manner that the posterior side of the optical body portion returns forward to decrease curvature thereof.

3. The accommodating intraocular lens as recited in claim 1,
wherein the optical portion and the support portion are configured such that the anterior optical plate portion of the optical portion and the anterior support plate portion of the support portion are integrally formed by a single plate member in a continuous manner, and the posterior optical plate portion of the optical portion and the posterior support plate portion of the support portion are integrally formed by a single plate member in a continuous manner.

4. The accommodating intraocular lens as recited in claim 1,
wherein the optical portion and the support portion are configured separately in such a manner that an outer peripheral portion of the anterior optical plate portion and an outer peripheral portion of the posterior optical plate portion of the optical portion are sandwiched between the anterior support plate portion of the support portion and the posterior support plate portion of the support portion.

5. The accommodating intraocular lens as recited in claim 1,
wherein the support portions are connected to each other along a circumferential direction by a deformable support portion connecting portion.

6. The accommodating intraocular lens as recited in claim 5,
wherein the support portion connecting portion is configured to be deformed in a curved shape protruding radially outward or radially inward when the optical portion and the support portion shift from a distance vision state to a near vision state to be reduced in diameter radially inward, and is configured to be deformed in a slightly curved shape extending in a peripheral direction or a linear shape when the optical portion and the support portion shift from a near vision state to a distance vision state to be increased in diameter radially outward.

7. The accommodating intraocular lens as recited in claim 1,
wherein the optical portion is provided with a fluid material filled therein and a filling port for filling the fluid material.

8. The accommodating intraocular lens as recited in claim 1,
wherein the optical portion is provided with a core member having rigidity at a central portion of the optical portion.

9. The accommodating intraocular lens as recited in claim 1, further comprising:
a support frame to be placed in the lens capsule,
wherein the support frame is provided with:
an anterior support frame portion to be arranged on an anterior side in the lens capsule to support the anterior capsule of the lens capsule;
a posterior support frame portion to be arranged on a posterior side in the lens capsule to support a posterior capsule of the lens capsule; and
one or a plurality of connecting portions connecting the anterior support frame portion and the posterior support frame portion, and
wherein the support frame is configured to accommodate the optical portion and the support portion therein.

10. The accommodating intraocular lens as recited in claim 1, further comprising:
a lens capsule expansion bag to be placed in the lens capsule,
wherein the lens capsule expansion bag has an elastic force,
wherein the lens capsule expansion bag is provided with:
an annular anterior bag portion to be placed in such a manner as to be in contact with the anterior capsule of the lens capsule;
an annular posterior bag portion to be placed in such a manner as to be in contact with the posterior capsule of the lens capsule; and
an annular intermediate bag portion protruded radially outward from outer peripheral portions of the anterior bag portion and the posterior bag portion, the annular intermediate bag portion being configured to be placed in such a manner as to be in contact with the equator of the lens capsule,
wherein the lens capsule expansion bag is configured to accommodate the optical portion and the support portion therein.

11. A method of producing the accommodating intraocular lens as recited in any one of claims 1 to 10, comprising the steps of:
preparing the support portion having a natural shape when the lens capsule is in a distance vision state and the optical portion having a natural shape when the lens capsule is in a near vision state;
forcibly reducing a diameter of the support portion radially inward as a whole such that the support portion shifts from the distance vision state to the near vision state;
joining the support portion and the optical portion while maintaining the near vision state of the support portion; and
deforming the optical portion from a state in which the curvature is large to a state in which the curvature is small by naturally expanding the support portion radially outward as a whole by an inherent restoring force of the support portion such that the support portion and the optical portion shift from the near vision state to the distance vision state.
